# EUROPEAN PATENT APPLICATION

(11) **EP 2 662 117 A1**
(43) Date of publication of application: **13.11.2013**
(21) Application number: 12167750.4
(22) Date of filing: 11.05.2012
(51) Int. Cl.: A61P 35/00, A61K 35/76, A61K 31/704, A61K 31/4412

(54) **Herpes Simplex Virus for the treatment of liver cancer**

(71) Applicant: Virttu Biologics Limited, Glasgow G51 4WF (GB)
(72) Inventor: Conner, Joe., Glasgow G51 4WF (GB)
(74) Representative: Clegg, Richard Ian

(57) **Abstract**

An oncolytic herpes simplex virus is disclosed for use in a method of treating primary liver cancer.

## Description

### Field of the Invention

The present invention relates to the use of an oncolytic herpes simplex virus in the treatment of primary liver cancer.

### Background to the Invention

Hepatocellular carcinoma (HCC), also known as malignant hepatoma, is a primary liver cancer and one of the most common solid organ malignancies. It is thought to be the third most common cause of death due to cancer causing an estimated 662,500 deaths annually. Diagnosed HCC tripled in the period between 1975 and 2005. Patient prognosis following diagnosis of HCC is poor, with ~90% patients not surviving beyond six months of diagnosis.

HCC is often associated with viral hepatitis B or C, alcoholic liver disease, cirrhosis, or haemochromatosis.

Existing treatments include surgery (e.g. hepatic resection or orthotopic liver transplantation), local ablative therapy (e.g. percutaneous ethanol injection), systemic chemotherapy (palliative), tumor embolisation and chemoembolistation (palliative).

Surgical resection represents the only realistic cure for HCC, although this procedure is limited by the nature of the tumor burden within the liver and the degree of liver function remaining after resection. Only about 10-15% of patients with HCC are suitable for surgical resection. Furthermore, instances of recurrence of tumor after surgical resection are high.

According to the EASL Panel of Experts on HCC "...none of the available options offers an unequivocal survival benefit to patients with intermediate-advanced HCC" (Bruix et al, 2001; J Hepatol 35:421-30).

Oncolytic virotherapy concerns the use of lytic viruses which selectively infect and kill cancer cells. Some oncolytic viruses are promising therapies as they display exquisite selection for replication in cancer cells and their self-limiting propagation within tumors results in fewer toxic side effects. Several oncolytic viruses have shown great promise in the clinic (Bell, J., Oncolytic Viruses: An Approved Product on the Horizon? Mol Ther. 2010; 18(2): 233-234).

Kelly et al and Geevarghese et al reported a Phase I/II trial of the safety and potential clinical efficacy of the HSV-1 strain F mutant NV1020 against liver metastasis of colorectal cancer (Kelly et al., Expert Opin Investig Drugs. 2008 July ; 17(7): 1105-1113; Geevarghese et al., HUMAN GENE THERAPY 21:1119-1128 (September 2010)). Patients enrolled in the trial had undergone partial hepatectomy and prior adjuvant chemotherapy (5-Fluorouracil (5-FU)). NV1020 was administered at doses of 3 x 10⁶, 1 x 10⁷, 3 x 10⁷, and 1 x 10⁸ by infusion to the hepatic artery. Patients received a chemotherapy infusion pump and cycles of floxuridine (FUDR) plus a continuation of the prior chemotherapy for 1 or 2 months after administration.

NV1020 is an attenuated, recombinant virus derived from the HSV-1 strain F mutant R7020, and was originally designed as a potential HSV-2 vaccine (Meignier B, Roizman B. Herpes simplex virus vaccines. Antiviral Res. 1985 Suppl 1:259-265; Meignier B, Longnecker R, Roizman B. In vivo behavior of genetically engineered herpes simplex viruses R7017 and R7020: construction and evaluation in rodents. J Infect Dis. 1988; 158(3):602-614).

NV1020 was attenuated by deletion of a 15-kb region at the UL/S junction encompassing only one copy of the diploid genes α0, α4, and γ₁34.5 encoding the proteins ICP0, ICP4, and ICP34.5, respectively, and one copy of UL56. NV1020 is further attenuated by a 700-bp deletion encompassing the thymidine kinase (TK) gene locus and the promoter for the gene UL24.

In addition to these deletions, NV1020 carries an exogenous inserted copy of the tk gene under restrictive control of the ICP4 promoter, and a 5.2-kb fragment of HSV-2 DNA. Both of these insertions are located at the UL/S junction (Wong RJ, Kim SH, Joe JK, et al. Effective treatment of head and neck squamous cell carcinoma by an oncolytic herpes simplex virus. J Am Coll Surg. 2001; 193(1):12-21).

### Summary of the Invention

In one aspect of the present invention an oncolytic herpes simplex virus is provided for use in a method of treating primary liver cancer, the method comprising intra-arterial or intravenous administration of an oncolytic herpes simplex virus.

In another aspect of the present invention a method of treating primary liver cancer is provided, the method comprising intra-arterial or intravenous administration of an oncolytic herpes simplex virus to a patient in need of treatment, thereby treating the primary liver cancer.

In another aspect of the present invention, the use of an oncolytic herpes simplex virus in the manufacture of a medicament for the treatment of primary liver cancer is provided, wherein the treatment involves intra-arterial or intravenous administration of an oncolytic herpes simplex virus.

In some embodiments the use, treatment or method of treatment further comprises administration of a chemotherapeutic agent, preferably doxorubicin and/or a kinase inhibitor, preferably sorafenib, and/or an anti-angiogenic, preferably bevacizumab and/or an EGFR inhibitor, preferably one or more of erlotinib, cetuximab or getifinib. In some embodiments the use, treatment or method of treatment further comprises simultaneous or sequential intra-arterial or intravenous administration of doxorubicin and/or oral administration of sorafenib. Administration of one or more, or each, of the oncolytic herpes simplex virus and doxorubicin may be to the hepatic artery or hepatic portal vein.

In another aspect of the present invention an oncolytic herpes simplex virus is provided for use in a method of treating primary liver cancer, wherein the method of treatment comprises administering doxorubicin and/or sorafenib to the patient in need of treatment.

In another aspect of the present invention doxorubicin and/or sorafenib is provided for use in a method of treating primary liver cancer, wherein the method of treatment comprises administering an oncolytic herpes simplex virus to the patient in need of treatment.

In another aspect of the present invention the use of an oncolytic herpes simplex virus in the manufacture of a medicament for use in a method of treatment of primary liver cancer is provided, wherein the method of treatment comprises administering doxorubicin and/or sorafenib to the patient in need of treatment.

In another aspect of the present invention the use of doxorubicin and/or sorafenib in the manufacture of a medicament for use in a method of treatment of primary liver cancer is provided, wherein the method of treatment comprises administering an oncolytic herpes simplex virus to the patient in need of treatment.

The primary liver cancer to be treated is preferably hepatocellular carcinoma.

In preferred embodiments all copies of the ICP34.5 gene in the genome of the oncolytic herpes simplex virus are modified such that the ICP34.5 gene is incapable of expressing a functional ICP34.5 gene product. As such the oncolytic herpes simplex virus may be an ICP34.5 null mutant.

In some embodiments one or both of the ICP34.5 genes in the genome of the oncolytic herpes simplex virus are modified such that the ICP34.5 gene is incapable of expressing a functional ICP34.5 gene product.

In some embodiments the oncolytic herpes simplex virus is a mutant of HSV-1 strain 17. In preferred embodiments the oncolytic herpes simplex virus is HSV1716 (ECACC Accession No. V92012803). In some embodiments the herpes simplex virus is a mutant of HSV-1 strain 17 mutant 1716.

In one aspect of the present invention HSV1716 for use in a method of treating hepatocellular carcinoma in a human patient is provided, the method comprising intra-arterial administration of HSV1716 to the hepatic artery. In some embodiments the method further comprises simultaneous or sequential intra-arterial administration of doxorubicin to the hepatic artery or oral administration of sorafenib.

In another aspect of the present invention a method of treating hepatocellular carcinoma in a human patient is provided, the method comprising intra-arterial administration of HSV1716 to the hepatic artery, thereby treating the hepatocellular carincoma. In some embodiments the method further comprises simultaneous or sequential intra-arterial administration of doxorubicin to the hepatic artery or oral administration of sorafenib.

In another aspect of the present invention, the use of HSV1716 in the manufacture of a medicament for the treatment of hepatocellular carcinoma in a human patient is provided, wherein the treatment involves intra-arterial administration of HSV1716 to the hepatic artery. In some embodiments the treatment further comprises simultaneous or sequential intra-arterial administration of doxorubicin to the hepatic artery or oral administration of sorafenib.

In another aspect of the present invention a pharmaceutical composition or medicament comprising an oncolytic herpes simplex virus and a chemotherapeutic agent is provided, wherein the chemotherapeutic agent is doxorubicin or sorafenib. In preferred embodiments the oncolytic herpes simplex virus is HSV1716.

In another aspect of the present invention a kit comprising a predetermined amount of oncolytic herpes simplex virus and a predetermined amount of chemotherapeutic agent is provided, wherein the chemotherapeutic agent is doxorubicin or sorafenib.

The kit may be provided together with instructions for the administration of the oncolytic herpes simplex virus, doxorubicin, and/or sorafenib sequentially or simultaneously in order to provide a treatment for primary liver cancer.

In another aspect of the present invention products containing therapeutically effective amounts of:
(i) an oncolytic herpes simplex virus, preferably HSV1716, and
(ii) Doxorubicin or Sorafenib
for simultaneous or sequential use in a method of medical treatment, preferably treatment of primary liver cancer, are provided. The products may be pharmaceutically acceptable formulations and may optionally be formulated as a combined preparation for coadministration.

### Description of Preferred Embodiments

### Oncolytic Herpes Simplex Virus

An oncolytic virus is a virus that will lyse cancer cells (oncolysis), preferably in a selective manner. Viruses that selectively replicate in dividing cells over non-dividing cells are often oncolytic. Oncolytic viruses are well known in the art and are reviewed in Molecular Therapy Vol.18 No.2 Feb 2010 pg 233-234.

The oncolytic herpes simplex virus may be any oncolytic herpes simplex virus. Preferably it is a replication-competent virus, being replication-competent at least in the target tumor cells.

The herpes simplex virus (HSV) genome comprises two covalently linked segments, designated long (L) and short (S). Each segment contains a unique sequence flanked by a pair of inverted terminal repeat sequences. The long repeat (RL or R_{L}) and the short repeat (RS or R_{S}) are distinct.

The HSV ICP34.5 (also called γ34.5) gene, which has been extensively studied, has been sequenced in HSV-1 strains F and syn17+ and in HSV-2 strain HG52. One copy of the ICP34.5 gene is located within each of the RL repeat regions. Mutants inactivating one or both copies of the ICP34.5 gene are known to lack neurovirulence, i.e. be avirulent/ non-neurovirulent (non-neurovirulence is defined by the ability to introduce a high titre of virus (approx 10⁶ plaque forming units (pfu)) to an animal or patient without causing a lethal encephalitis such that the LD₅₀ in animals, e.g. mice, or human patients is in the approximate range of ≥10⁶ pfu), and be oncolytic.

Oncolytic HSV that may be used in the present invention include HSV in which one or both of the γ34.5 (also called ICP34.5) genes are modified (e.g. by mutation which may be a deletion, insertion, addition or substitution) such that the respective gene is incapable of expressing, e.g. encoding, a functional ICP34.5 protein. Most preferably, in HSV according to the invention both copies of the γ34.5 gene are modified such that the modified HSV is not capable of expressing, e.g. producing, a functional ICP34.5 protein.

Accordingly, in preferred embodiments the oncolytic herpes simplex virus may be an ICP34.5 null mutant where all copies of the ICP34.5 gene present in the herpes simplex virus genome (two copies are normally present) are disrupted such that the herpes simplex virus is incapable of producing a functional ICP34.5 gene product.

In other embodiments the oncolytic herpes simplex virus may lack at least one expressible ICP34.5 gene. In some embodiments the herpes simplex virus may lack only one expressible ICP34.5 gene. In other embodiments the herpes simplex virus may lack both expressible ICP34.5 genes. In still other embodiments each ICP34.5 gene present in the herpes simplex virus may not be expressible. Lack of an expressible ICP34.5 gene means, for example, that expression of the ICP34.5 gene does not result in a functional ICP34.5 gene product.

Oncolytic herpes simplex virus may be derived from any HSV including any laboratory strain or clinical isolate (non-laboratory strain) of HSV. In some preferred embodiments the HSV is a mutant of HSV-1 or HSV-2. Alternatively the HSV may be an intertypic recombinant of HSV-1 and HSV-2. The mutant may be of one of laboratory strains HSV-1 strain 17, HSV-1 strain F or HSV-2 strain HG52. The mutant may be of the non-laboratory strain JS-1. Preferably the mutant is a mutant of HSV-1 strain 17. The herpes simplex virus may be one of HSV-1 strain 17 mutant 1716, HSV-1 strain F mutant R3616, HSV-1 strain F mutant G207, HSV-1 mutant NV1020, or a further mutant thereof in which the HSV genome contains additional mutations and/or one or more heterologous nucleotide sequences. Additional mutations may include disabling mutations, which may affect the virulence of the virus or its ability to replicate. For example, mutations may be made in any one or more of ICP6, ICP0, ICP4, ICP27. Preferably, a mutation in one of these genes (optionally in both copies of the gene where appropriate) leads to an inability (or reduction of the ability) of the HSV to express the corresponding functional polypeptide. By way of example, the additional mutation of the HSV genome may be accomplished by addition, deletion, insertion or substitution of nucleotides.

A number of oncolytic herpes simplex viruses are known in the art. Examples include HSV1716, R3616 (e.g. see Chou & Roizman, Proc. Natl. Acad. Sci. Vol.89, pp.3266-3270, April 1992), G207 (Toda et al, Human Gene Therapy 9:2177-2185, October 10, 1995), NV1020 (Geevarghese et al, Human Gene Therapy 2010 Sep; 21 (9):1119-28), RE6 (Thompson et al, Virology 131, 171-179 (1983)), and Oncovex™ (Simpson et al, Cancer Res 2006; 66:(9) 4835-4842 May 1, 2006; Liu et al, Gene Therapy (2003): 10, 292-303).

In some preferred embodiments the herpes simplex virus is HSV-1 strain 17 mutant 1716 (HSV1716). HSV 1716 is an oncolytic, non-neurovirulent HSV and is described in EP 0571410, WO 92/13943, Brown et al (Journal of General Virology (1994), 75, 2367-2377) and MacLean et al (Journal of General Virology (1991), 72, 631-639). HSV 1716 has been deposited on 28 January 1992 at the European Collection of Animal Cell Cultures, Vaccine Research and Production Laboratories, Public Health Laboratory Services, Porton Down, Salisbury, Wiltshire, SP4 0JG, United Kingdom under accession number V92012803 in accordance with the provisions of the Budapest Treaty on the International Recognition of the Deposit of Microorganisms for the Purposes of Patent Procedure (herein referred to as the 'Budapest Treaty').

In some embodiments the herpes simplex virus is a mutant of HSV-1 strain 17 modified such that both ICP34.5 genes do not express a functional gene product, e.g. by mutation (e.g. insertion, deletion, addition, substitution) of the ICP34.5 gene, but otherwise resembling or substantially resembling the genome of the wild type parent virus HSV-1 strain 17+. That is, the virus may be a variant of HSV1716, having a genome mutated so as to inactivate both copies of the ICP34.5 gene of HSV-1 strain 17+ but not otherwise altered to insert or delete/modify other protein coding sequences.

In some embodiments the genome of an oncolytic virus according to the present invention may be further modified to contain nucleic acid encoding at least one copy of a polypeptide that is heterologous to the virus (i.e. is not normally found in wild type virus) such that the polypeptide can be expressed from the nucleic acid. As such, the oncolytic virus may also be an expression vector from which the polypeptide may be expressed. Examples of such viruses are described in WO2005/049846 and WO2005/049845.

In order to effect expression of the polypeptide, nucleic acid encoding the polypeptide is preferably operably linked to a regulatory sequence, e.g. a promoter, capable of effecting transcription of the nucleic acid encoding the polypeptide. A regulatory sequence (e.g. promoter) that is operably linked to a nucleotide sequence may be located adjacent to that sequence or in close proximity such that the regulatory sequence can effect and/or control expression of a product of the nucleotide sequence. The encoded product of the nucleotide sequence may therefore be expressible from that regulatory sequence.

Oncolytic viruses may be formulated as medicaments and pharmaceutical compositions for clinical use and in such formulations may be combined with a pharmaceutically acceptable carrier, diluent or adjuvant. The composition may be formulated for intravenous or, intra-arterial routes of administration, which preferably include injection. Suitable formulations may comprise the virus in a sterile or isotonic medium. Medicaments and pharmaceutical compositions may be formulated in fluid (including gel) or solid (e.g. tablet) form. Fluid formulations may be formulated for administration by injection or via catheter to a selected region of the human or animal body. Medicaments and pharmaceutical compositions may be formulated in combination with one or more other active agents, e.g. a chemotherapeutic agent such as doxorubicin or sorafenib and/or an embolization agent and/or a contrast agent (e.g. Lipiodol).

Administration is preferably in a "therapeutically effective amount", this being sufficient to show benefit to the individual. The actual amount administered, and rate and time-course of administration, will depend on the nature and severity of the disease being treated. Prescription of treatment, e.g. decisions on dosage etc, is within the responsibility of general practitioners and other medical doctors, and typically takes account of the disorder to be treated, the condition of the individual patient, the site of delivery, the method of administration and other factors known to practitioners. Examples of the techniques and protocols mentioned above can be found in Remington's Pharmaceutical Sciences, 20th Edition, 2000, pub. Lippincott, Williams & Wilkins.

Targeting therapies may be used to deliver the oncolytic virus to certain types of cell, e.g. by the use of targeting systems such as antibody or cell specific ligands. Targeting may be desirable for a variety of reasons; for example if the virus is unacceptably toxic in high dose, or if it would otherwise require too high a dosage, or if it would not otherwise be able to enter the target cells.

HSV capable of targeting cells and tissues are described in (PCT/GB2003/000603; WO 03/068809), hereby incorporated in its entirety by reference.

An oncolytic virus may be administered alone or in combination with other treatments, either simultaneously or sequentially dependent upon the condition to be treated. Such other treatments may include chemotherapy (including either systemic treatment with a chemotherapeutic agent or targeted therapy using small molecule or biological molecule (e.g. antibody) based agents that target key pathways in tumor development, maintenance or progression) or radiotherapy provided to the subject as a standard of care for treatment of the cancer.

Oncolytic herpes simplex virus may be administered in any therapeutically effective dosage amount. The inventors have identified that low dose of virus (~<10⁶ pfu) is as effective in treating primary liver cancer as higher doses (~>10⁷ pfu). In some embodiments a low dose of oncolytic herpes simplex virus is preferred, e.g. less than 1x10⁶ pfu, less than 1x10⁵ pfu or less than 1x10⁴ pfu.

### Cancer

A cancer may be any unwanted cell proliferation (or any disease manifesting itself by unwanted cell proliferation), neoplasm or tumor or increased risk of or predisposition to the unwanted cell proliferation, neoplasm or tumor. The cancer may be benign or malignant. A neoplasm or tumor may be any abnormal growth or proliferation of cells and may be located in any tissue.

The present invention is concerned with the treatment of primary liver cancer, i.e. a cancer originating in the liver. Examples of primary liver cancer include hepatocellular carcinoma (also called hepatoma), biliary tree cancer, cholangiocarcinoma (bile duct cancer) and gallbladder cancer.

In some embodiments the primary liver cancer is an unresectable liver cancer, e.g. unresectable hepatocellular carcinom. Determination of whether or not a primary liver cancer is resectable is within the skill and knowledge of a medical practitioner or surgeon working in the field of liver cancer.

### Subjects

The subject to be treated may be any animal or human. The subject is preferably mammalian, more preferably human. The subject may be a non-human mammal, but is more preferably human. The subject may be male or female. The subject may be a patient. A subject may have been diagnosed with a primary liver cancer, or be suspected of having a primary liver cancer prior to diagnosis.

### Chemotherapy

Chemotherapy refers to treatment of a tumor with a drug. For example, the drug may be a chemical entity, e.g. small molecule pharmaceutical, antibiotic, DNA intercalator, protein inhibitor (e.g. kinase inhibitor) or a biological agent, e.g. antibody, antibody fragment, nucleic acid or peptide aptamer, nucleic acid (e.g. DNA, RNA), peptide, polypeptide, or protein. The drug may be formulated as a pharmaceutical composition or medicament. The formulation may comprise one or more drugs (e.g. one or more active agents) together with one or more pharmaceutically acceptable diluents, excipients or carriers.

A treatment may involve administration of more than one drug. A drug may be administered alone or in combination with other treatments, either simultaneously or sequentially dependent upon the condition to be treated. For example, the chemotherapy may be a co-therapy involving administration of two drugs/agents, one or more of which may be intended to treat the tumor. In the present invention an oncolytic herpes simplex virus and chemotherapeutic may be administered simultaneously, separately, or sequentially which may allow the two agents be present in the tumor requiring treatment at the same time and thereby provide a combined therapeutic effect, which may be additive or synergistic.

The chemotherapy may be administered by one or more routes of administration, e.g. parenteral, intra-arterial injection or infusion, intravenous injection or infusion, intraperitoneal, intratumoral or oral. Administration is preferably in a "therapeutically effective amount", this being sufficient to show benefit to the individual. The actual amount administered, and rate and time-course of administration, will depend on the nature and severity of the disease being treated. Prescription of treatment, e.g. decisions on dosage etc, is within the responsibility of general practitioners and other medical doctors, and typically takes account of the disorder to be treated, the condition of the individual patient, the site of delivery, the method of administration and other factors known to practitioners. Examples of the techniques and protocols mentioned above can be found in Remington's Pharmaceutical Sciences, 20th Edition, 2000, pub. Lippincott, Williams & Wilkins.

The chemotherapy may be administered according to a treatment regime. The treatment regime may be a pre-determined timetable, plan, scheme or schedule of chemotherapy administration which may be prepared by a physician or medical practitioner and may be tailored to suit the patient requiring treatment.

The treatment regime may indicate one or more of: the type of chemotherapy to administer to the patient; the dose of each drug; the time interval between administrations; the length of each treatment; the number and nature of any treatment holidays, if any etc. For a co-therapy a single treatment regime may be provided which indicates how each drug/agent is to be administered.

### Chemotherapeutic agents

Chemotherapeutic agents known for use in treating primary liver cancer include 5-Fluorouracil, floxuridine (FUDR), leucovorin, oxaliplatin, irinotecan, doxorubicin and sorafenib.

Some chemotherapeutic agents may be administered by hepatic arterial infusion (HAI) which allows for delivery directly to the liver.

Doxorubicin ((7S,9S)-7-[(2R,4S,5S,6S)-4-amino-5-hydroxy-6-methyloxan-2-yl]oxy-6,9,11-trihydroxy-9-(2-hydroxyacetyl)-4-methoxy-8,10-dihydro-7H-tetracene-5,12-dione), marketed under the trade name Adriamycin™ (amongst others). Doxorubicin is an anthracycline antibiotic and DNA intercalator.

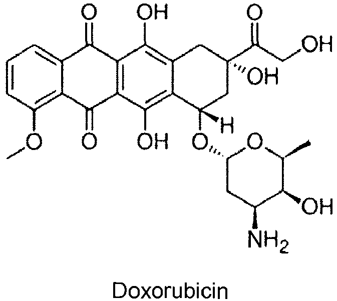

Typical dosages of doxorubicin for treatment of human patients may be in the range 40-60mg/m² (iv).

Sorafenib (4-[4-[[4-chloro-3-(trifluoromethyl)phenyl]carbamoylamino]phenoxy]-N-methylpyridine-2-carboxamide), marketed under the trade name Nexavar™, is a tyrosine kinase and Raf kinase inhibitor and targets the Raf/Mek/Erk (MAP Kinase) pathway.

Sorafenib has received EU marketing authorisation and FDA approval for the treatment of HCC.

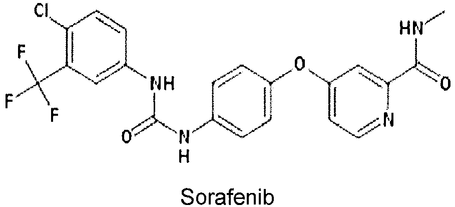

Typical dosages of sorafenib for treatment of human patients may be about 200mg or 400mg per day (oral) or about 5-20mg/kg (iv).

In this specification reference to doxorubicin and sorafenib includes their salts (e.g. hydrochloride or tosylate salts), hydrates, prodrug formulations and other pharmaceutically acceptable formulations that provide the active doxorubicin or sorafenib agent when administered to the human or mammalian body.

### Forms of Chemotherapeutic Agent

The active compound of a given chemotherapeutic agent may be provided in the form of a corresponding salt, solvate, or prodrug. In this specification reference to the chemotherapeutic agent includes reference to such forms.

### Salts

It may be convenient or desirable to prepare, purify, and/or handle a corresponding salt of the active compound, for example, a pharmaceutically-acceptable salt. Examples of pharmaceutically acceptable salts are discussed in Berge et al., 1977, "Pharmaceutically Acceptable Salts," J. Pharm. Sci., Vol. 66, pp. 1-19.

For example, if the compound is anionic, or has a functional group which may be anionic (e.g., -COOH may be -COO⁻), then a salt may be formed with a suitable cation. Examples of suitable inorganic cations include, but are not limited to, alkali metal ions such as Na⁺ and K⁺, alkaline earth cations such as Ca²⁺ and Mg²⁺, and other cations such as Al⁺³. Examples of suitable organic cations include, but are not limited to, ammonium ion (i.e., NH₄⁺) and substituted ammonium ions (e.g., NH₃R⁺, NH₂R₂⁺, NHR₃⁺, NR₄⁺). Examples of some suitable substituted ammonium ions are those derived from: ethylamine, diethylamine, dicyclohexylamine, triethylamine, butylamine, ethylenediamine, ethanolamine, diethanolamine, piperazine, benzylamine, phenylbenzylamine, choline, meglumine, and tromethamine, as well as amino acids, such as lysine and arginine. An example of a common quaternary ammonium ion is N(CH₃)₄⁺.

If the compound is cationic, or has a functional group which may be cationic (e.g., -NH₂ may be -NH₃⁺), then a salt may be formed with a suitable anion. Examples of suitable inorganic anions include, but are not limited to, those derived from the following inorganic acids: hydrochloric, hydrobromic, hydroiodic, sulfuric, sulfurous, nitric, nitrous, phosphoric, and phosphorous.

Examples of suitable organic anions include, but are not limited to, those derived from the following organic acids: 2-acetyoxybenzoic, acetic, ascorbic, aspartic, benzoic, camphorsulfonic, cinnamic, citric, edetic, ethanedisulfonic, ethanesulfonic, fumaric, glucheptonic, gluconic, glutamic, glycolic, hydroxymaleic, hydroxynaphthalene carboxylic, isethionic, lactic, lactobionic, lauric, maleic, malic, methanesulfonic, mucic, oleic, oxalic, palmitic, pamoic, pantothenic, phenylacetic, phenylsulfonic, propionic, pyruvic, salicylic, stearic, succinic, sulfanilic, tartaric, toluenesulfonic, and valeric. Examples of suitable polymeric organic anions include, but are not limited to, those derived from the following polymeric acids: tannic acid, carboxymethyl cellulose.

Unless otherwise specified, a reference to a particular compound also include salt forms thereof.

### Solvates

It may be convenient or desirable to prepare, purify, and/or handle a corresponding solvate of the active compound. The term "solvate" is used herein in the conventional sense to refer to a complex of solute (e.g., active compound, salt of active compound) and solvent. If the solvent is water, the solvate may be conveniently referred to as a hydrate, for example, a mono-hydrate, a di-hydrate, a tri-hydrate, etc.

Unless otherwise specified, a reference to a particular compound also include solvate forms thereof.

### Prodrugs

It may be convenient or desirable to prepare, purify, and/or handle the active compound in the form of a prodrug. The term "prodrug," as used herein, pertains to a compound which, when metabolised (e.g., in vivo), yields the desired active compound. Typically, the prodrug is inactive, or less active than the active compound, but may provide advantageous handling, administration, or metabolic properties.

Unless otherwise specified, a reference to a particular compound also include prodrugs thereof.

For example, some prodrugs are esters of the active compound (e.g., a physiologically acceptable metabolically labile ester). During metabolism, the ester group (-C(=O)OR) is cleaved to yield the active drug. Such esters may be formed by esterification, for example, of any of the carboxylic acid groups (-C(=O)OH) in the parent compound, with, where appropriate, prior protection of any other reactive groups present in the parent compound, followed by deprotection if required.

Examples of such metabolically labile esters include those of the formula -C(=O)OR wherein R is: C₁₋₇alkyl (e.g., -Me, -Et, -nPr, -iPr, -nBu, -sBu, -iBu, -tBu); C₁₋₇aminoalkyl (e.g., aminoethyl; 2-(N,N-diethylamino)ethyl; 2-(4-morpholino)ethyl); and acyloxy-C₁₋₇alkyl (e.g., acyloxymethyl; acyloxyethyl; pivaloyloxymethyl; acetoxymethyl; 1-acetoxyethyl; 1-(1-methoxy-1-methyl)ethyl-carbonxyloxyethyl; 1-(benzoyloxy)ethyl; isopropoxycarbonyloxymethyl; 1-isopropoxy-carbonyloxyethyl; cyclohexyl-carbonyloxymethyl; 1-cyclohexyl-carbonyloxyethyl; cyclohexyloxy-carbonyloxymethyl; 1-cyclohexyloxycarbonyloxyethyl; (4-tetrahydropyranyloxy) carbonyloxymethyl; 1-(4-tetrahydropyranyloxy)carbonyloxyethyl; (4-tetrahydropyranyl)carbonyloxymethyl; and 1-(4-tetrahydropyranyl)carbonyloxyethyl).

Also, some prodrugs are activated enzymatically to yield the active compound, or a compound which, upon further chemical reaction, yields the active compound (for example, as in ADEPT, GDEPT, LIDEPT, etc.). For example, the prodrug may be a sugar derivative or other glycoside conjugate, or may be an amino acid ester derivative.

### Other Chemotherapeutic Agents

In addition to treating a cancer by using an oncolytic herpes simplex virus optionally in combination with doxorubicin or sorafenib, subjects being treated may also receive treatment with other chemotherapeutic agents. For example, other chemotherapeutic agents may be selected from:
(i) alkylating agents such as cisplatin, carboplatin, mechlorethamine, cyclophosphamide, chlorambucil, ifosfamide;
(ii) purine or pyrimidine anti-metabolites such as azathiopurine or mercaptopurine;
(iii) alkaloids and terpenoids, such as vinca alkaloids (e.g. vincristine, vinblastine, vinorelbine, vindesine), podophyllotoxin, etoposide, teniposide, taxanes such as paclitaxel (Taxol™), docetaxel;
(iv) topoisomerase inhibitors such as the type I topoisomerase inhibitors camptothecins irinotecan and topotecan, or the type II topoisomerase inhibitors amsacrine, etoposide, etoposide phosphate, teniposide;
(v) antitumor antibiotics (e.g. anthracyline antibiotics) such as dactinomycin, doxorubicin (Adriamycin™), epirubicin, bleomycin, rapamycin;
(vi) antibody based agents, such as anti-VEGF, anti-TNFα, anti-IL-2, antiGpIIb/IIIa, anti-CD-52, anti-CD20, anti-RSV, anti-HER2/neu(erbB2), anti-TNF receptor, anti-EGFR antibodies, monoclonal antibodies or antibody fragments, examples include: cetuximab, panitumumab, infliximab, basiliximab, bevacizumab (Avastin®), abciximab, daclizumab, gemtuzumab, alemtuzumab, rituximab (Mabthera®), palivizumab, trastuzumab, etanercept, adalimumab, nimotuzumab,
(vii) EGFR inihibitors such as erlotinib, cetuximab and gefitinib,
(viii) anti-angiogenic agents such as bevacizumab (Avastin®).

### Simultaneous or Sequential Administration

Compositions may be administered alone or in combination with other treatments, either simultaneously or sequentially dependent upon the condition to be treated.

In this specification an oncolytic herpes simplex virus and chemotherapeutic agent may be administered simultaneously or sequentially.

Simultaneous administration refers to administration of the oncolytic herpes simplex virus and chemotherapeutic agent together, for example as a pharmaceutical composition containing both agents, or immediately after each other via the same route of administration, e.g. to the same artery, vein or other blood vessel.

Sequential administration refers to administration of one of the oncolytic herpes simplex virus or chemotherapeutic agent followed after a given time interval by separate administration of the other agent. It is not required that the two agents are administered by the same route, although in some embodiments this preferred. For example, in some embodiments one of the oncolytic herpes simplex virus or chemotherapeutic agent is administered by intra-arterial or intravenous administration followed, after a predetermined time interval, by administration of the other agent to the same artery or vein, preferably to the same site, e.g. by re-use of a catheter or tube inserted in the patient's artery or vein. The predetermined time interval may be any time interval.

Whilst simultaneous or sequential administration is intended such that both the oncolytic herpes simplex virus and chemotherapeutic agent are delivered to the same tumor tissue to effect treatment it is not essential for both agents to be present in the tumor tissue in active form at the same time.

However, in some embodiments of sequential administration the time interval is selected such that the oncolytic herpes simplex virus and chemotherapeutic agent are expected to be present in the tumor tissue in active form at the same time, thereby allowing for a combined, additive or synergistic effect of the two agents in treating the tumor. In such embodiments the time interval selected may be any one of 5 minutes or less, 10 minutes or less, 15 minutes or less, 20 minutes or less, 25 minutes or less, 30 minutes or less, 45 minutes or less, 60 minutes or less, 90 minutes or less, 120 minutes or less, 180 minutes or less, 240 minutes or less, 300 minutes or less, 360 minutes or less, or 720 minutes or less, or 1 day or less, or 2 days or less.

### Intra-arterial administration

Infusion of active agents (e.g. oncolytic herpes simplex virus and/or chemotherapeutic agent(s)) to the hepatic artery has been developed as a means of directly delivering therapeutic and imaging/diagnostic agents to the liver.

At its simplest, a catheter is inserted to the patient's vasculature and is manipulated to gain access to the hepatic artery. Active agents may then be administered to the blood flowing directly to the liver. Where more than one agent is to be delivered, the agents may be administered together, i.e. in combination, or separately but over a period of time that allows for both agents to be present in the liver at the same time and thereby allow for them to exert a combined or synergistic effect.

In practice, intra-arterial infusion is preferred, although in principle a similar infusion technique could be applied to deliver agents to the liver via the hepatic portal vein.

One technique developed for intra-arterial infusion suitable in the context of treatment of primary liver cancer is trans-arterial chemoembolization (TACE).

TACE is normally performed by an interventional radiologist and involves accessing the hepatic artery with a catheter, which is possible by puncturing the common femoral artery in the right groin and passing a catheter through the abdominal aorta, through the celiac trunk and common hepatic artery, into the proper hepatic artery.

An arteriogram is performed to identify the branches of the hepatic artery supplying the tumor(s). Smaller catheters may then be threaded into these branches (so-called superselective positioning). This allows precision delivery of the active agents to the tumor tissue.

Once the catheter is in position, doses of the active agent (e.g. oncolytic herpes simplex virus, and/or chemotherapeutic agent and/or embolisation agent and/or contrast agent) are injected through the catheter. The total dose may be given to a single vessel, or if there are several tumor foci may be divided among several vessels supplying the tumors. Because most tumors are supplied by the hepatic artery, arterial embolization interrupts the blood supply to the tumor and delays tumor growth. The focused nature of the administration of active agents enables delivery of a high therapeutic dose to the tissue requiring treatment whilst reducing systemic exposure and therefore toxicity. Embolization of the vessel assists this process in that the active agent(s) is not washed out from the tumor bed and the supply of nutrients to the tumor is decreased thereby promoting tumor necrosis.

TACE is widely used as a palliative treatment for surgically unresectable primary or metastatic HCC tumors.

### Other Treatments

Administration of an oncolytic herpes simplex virus and optionally chemotherapeutic agent in accordance with the present invention may be combined with other medical procedures such surgical resection of the tumor or embolization (e.g. chemical embolization) of the tumor.

### Dosage regime

The inventors have identified that multiple doses of oncolytic herpes simplex virus provide greater efficacy in reducing tumor volume and tumor remission. Accordingly, multiple doses of the oncolytic virus may be provided. One or more, or each, of the doses may be accompanied by simultaneous or sequential administration of a chemotherapeutic agent.

Multiple doses may be separated by a predetermined time interval, which may be selected to be one of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, or 31 days, or 1, 2, 3, 4, 5, or 6 months.

By way of example, doses may be given once every 7, 14, 21 or 28 days (plus or minus 3, 2, or 1 days). The dose of oncolytic herpes simplex virus given at each dosing point may be the same, but this is not essential. For example, it may be appropriate to give a higher priming dose at the first, second and/or third dosing points.

### Kits

In some aspects of the present invention a kit of parts is provided. In some embodiments the kit may have at least one container having a predetermined quantity of oncolytic herpes simplex virus, e.g. predetermined viral dose or number/quantity/concentration of viral particles. The oncolytic herpes simplex virus may be formulated so as to be suitable for injection or infusion to the blood. In some embodiments the kit may further comprise at least one container having a predetermined quantity of chemotherapeutic agent, e.g. doxorubicin or sorafenib. The chemotherapeutic agent may also be formulated so as to be suitable for injection or infusion to the blood. In some embodiments a container having a mixture of a predetermined quantity of oncolytic herpes simplex virus and predetermined quantity of chemotherapeutic agent is provided, which may optionally be formulated so as to be suitable for injection or infusion to the blood.

In some embodiments the kit may also contain apparatus suitable to administer one or more doses of the oncolytic herpes simplex virus and/or chemotherapeutic agent. Such apparatus may include one or more of a catheter and/or needle and/or syringe, such apparatus preferably being provided in sterile form.

The kit may further comprise instructions for the administration of a therapeutically effective dose of the oncolytic herpes simplex virus and/or chemotherapeutic agent. The instructions may include instructions for administration to the hepatic artery or hepatic portal vein, or for oral administration.

The invention includes the combination of the aspects and preferred features described except where such a combination is clearly impermissible or expressly avoided.

The section headings used herein are for organizational purposes only and are not to be construed as limiting the subject matter described.

Aspects and embodiments of the present invention will now be illustrated, by way of example, with reference to the accompanying figures. Further aspects and embodiments will be apparent to those skilled in the art. All documents mentioned in this text are incorporated herein by reference.

### Brief Description of the Figures

Embodiments and experiments illustrating the principles of the invention will now be discussed with reference to the accompanying figures in which:
**Figure 1****.** Graph showing yields from Huh-7 cells 72 hours after infection with different multiplicities of infection of HSV1716 or HSV-1 17+.
**Figure 2****.** Graph showing yields from Huh-7 cells at 24, 48 and 72 hours after infection with HSV-1 17+ or HSV1716 at various multiplicities of infection.
**Figure 3****.** Graph showing replication of HSV-1 17+ and HSV1716 in HepG2-luc2 cells, showing virus titres at 48 and 72 hours after infection of HepG2-luc2 cells with HSV-1 17+ and HSV1716 at multiplicities of infection of 0.1 or 0.01.
**Figure 4****.** Graph showing HepG2-luc2 luciferase activity (luminosity) at 24 hours and 48 hours post infection with HSV-1 17+ and HSV1716 at multiplicities of infection of 1 or 0.1.

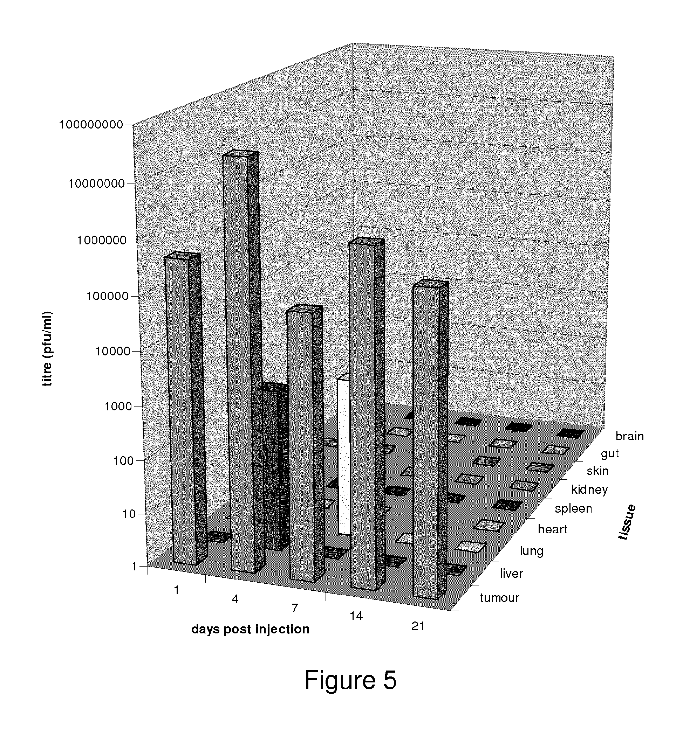

**Figure 5****.** Chart showing biodistribution by titre in nude mice bearing Huh-7 xenografts following intravenous injection of HSV1716.
**Figure 6****.** Graph showing growth of HuH-7 tumor xenografts as far as time at which no virus controls were sacrificed. Mice were exposed to no virus, or HSV1716 at 1x10⁶ pfu or 1x10⁷ pfu, administered by tail vein injection on days 1 and 4.
**Figure 7****.** Graph showing growth of HuH-7 tumor xenografts as far as end of the experiment. Mice were exposed to no virus, or HSV1716 at 1x10⁶ pfu or 1x10⁷ pfu, administered by tail vein injection on days 1 and 4.
**Figure 8****.** Graph showing survival of mice having HuH-7 xenografts following treatment with no virus, or HSV1716 at 1x10⁶ pfu or 1x10⁷ pfu, administered by tail vein injection on days 1 and 4.

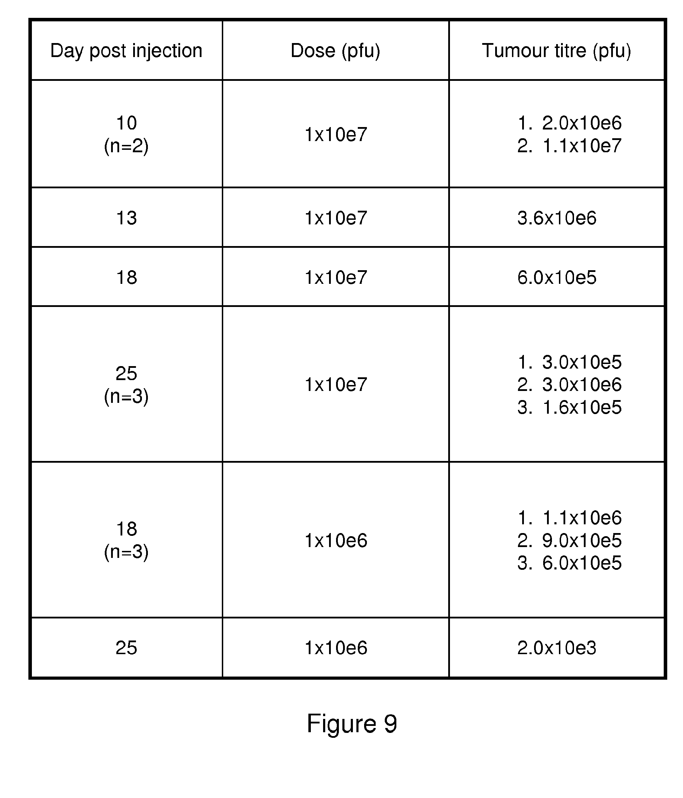

**Figure 9****.** Table showing titration of HSV1716 in HuH-7 tumor extracts.
**Figure 10****.** Graph showing survival of mice having HuH-7 xenografts following treatment with no virus, or HSV1716 at 1x10⁵ pfu, 1x10⁶ pfu or 1x10⁷ pfu, administered by tail vein injection on days 1, 14 and 29.
**Figure 11****.** Graph showing growth of HuH-7 tumor xenografts as far as end of the experiment. Mice were exposed to no virus, or HSV1716 at 1x10⁵ pfu, 1x10⁶ pfu or or 1x10⁷ pfu, administered by tail vein injection on days 1, 14 and 29.

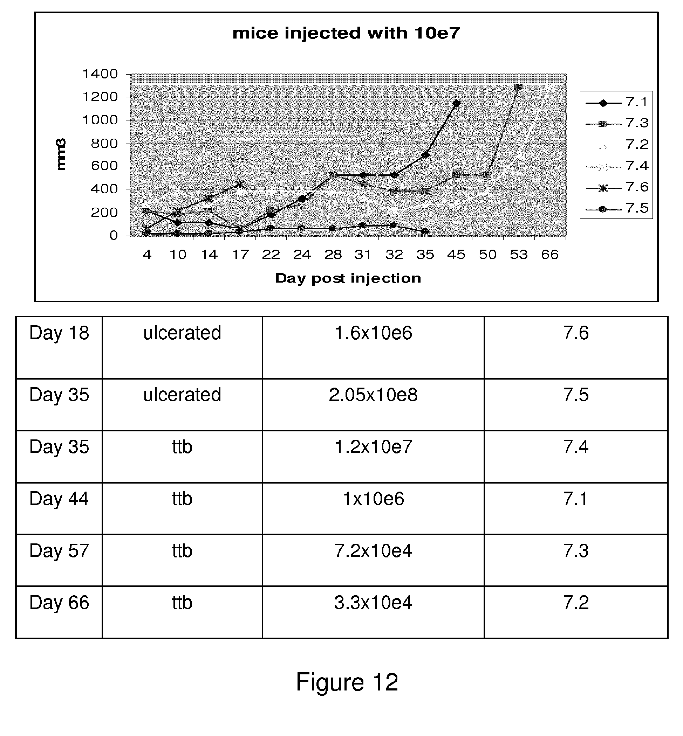

**Figure 12****.** Graph showing growth of tumor and table showing intratumoral viral titre in individual mice treated with 1x10⁷ pfu HSV1716 by tail vein injection on days 1, 14 and 29.

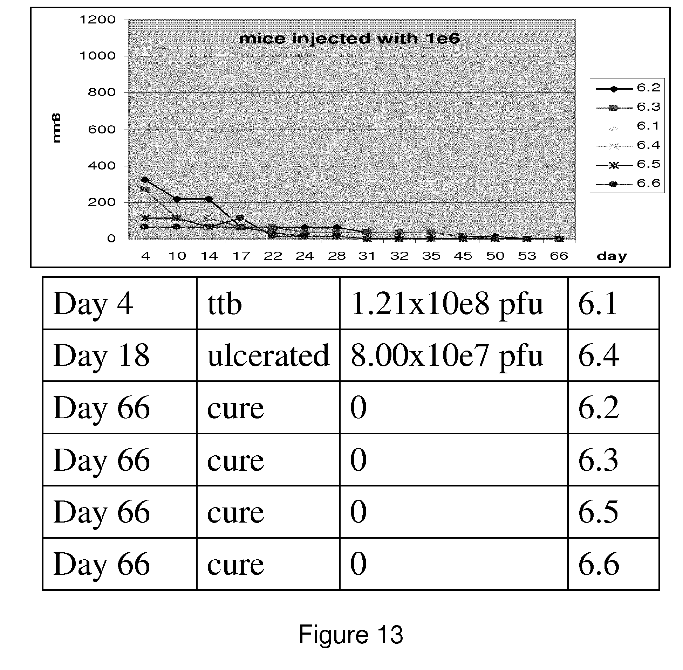

**Figure 13****.** Graph showing growth of tumor and table showing intratumoral viral titre in individual mice treated with 1x10⁶ pfu HSV1716 by tail vein injection on days 1, 14 and 29.

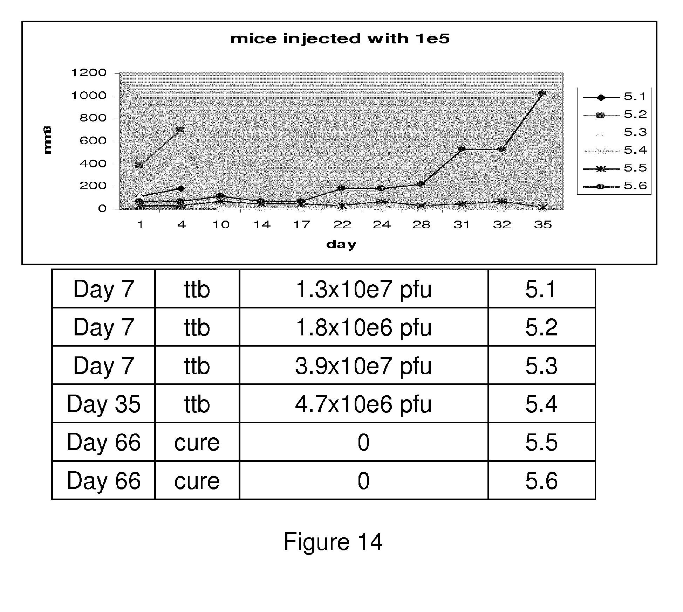

**Figure 14****.** Graph showing growth of tumor and table showing intratumoral viral titre in individual mice treated with 1x10⁵ pfu HSV1716 by tail vein injection on days 1, 14 and 29.
**Figure 15****.** Table and Graph showing infection of HuH-7 cells in tumor xenografts by HSV1716 after intravenous administration.
**Figure 16****.** Table showing infection of HuH-7 cells in tumor xenografts by HSV1716 after intravenous administration of varying doses of HSV1716.
**Figure 17****.** Chart showing HSV1716 localisation to HuH-7 mouse xenografts.
**Figure 18****.** Chart showing biodistribution by luciferase activity in nude mice bearing HuH-7 xenografts following intravenous injection of HSV1716luc.

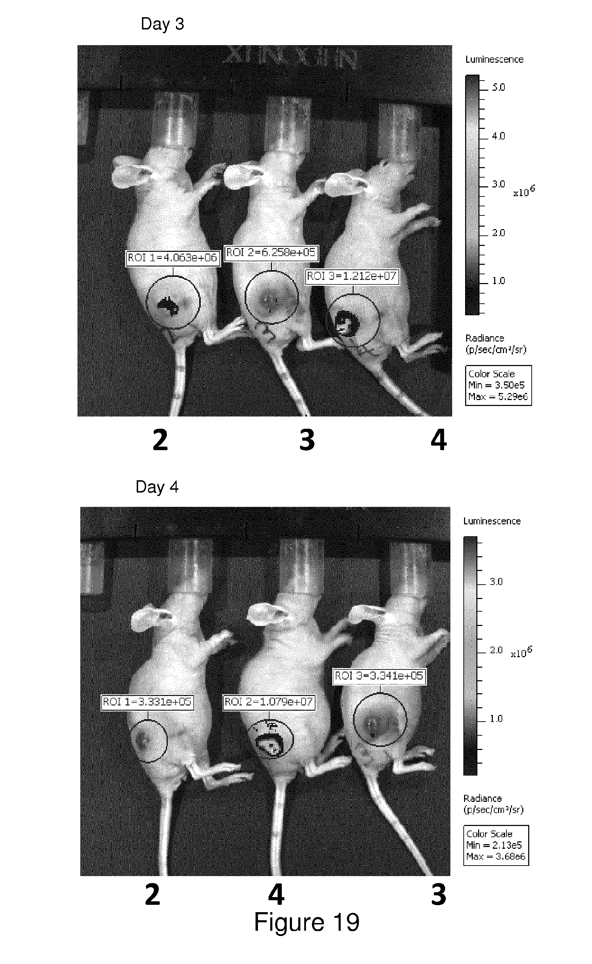

**Figure 19****.** Photographs showing luciferase expression in "dox 1" treated nude mice at day 3 and day 4 following intratumoral injection.

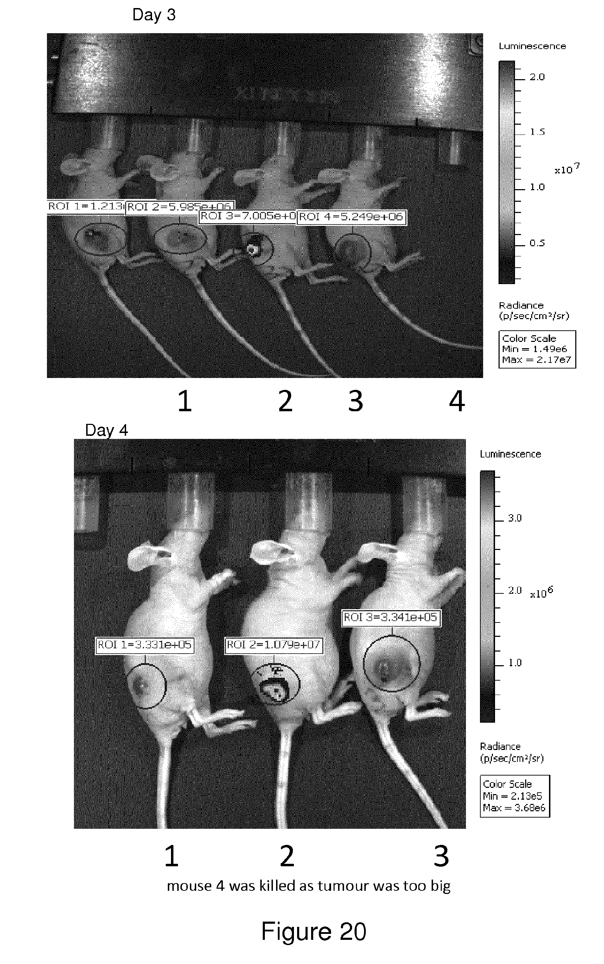

**Figure 20****.** Photographs showing luciferase expression in "dox 2" treated nude mice at day 3 and day 4 following intratumoral injection. NB by day 4 mouse 4 was killed because the tumor was too large.
**Figure 21****.** Charts showing light emissions of "dox 1" and "dox 2" treated mice at days 3, 4, 11 and 14 following intratumoral injection.

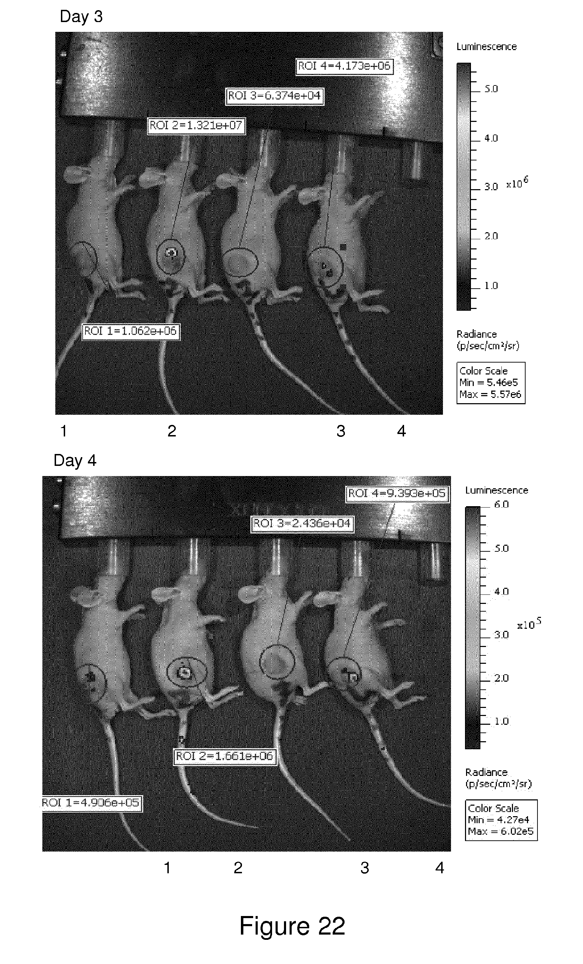

**Figure 22****.** Photographs showing luciferase expression in "dox 4" treated nude mice at day 3 and day 4 following administration.

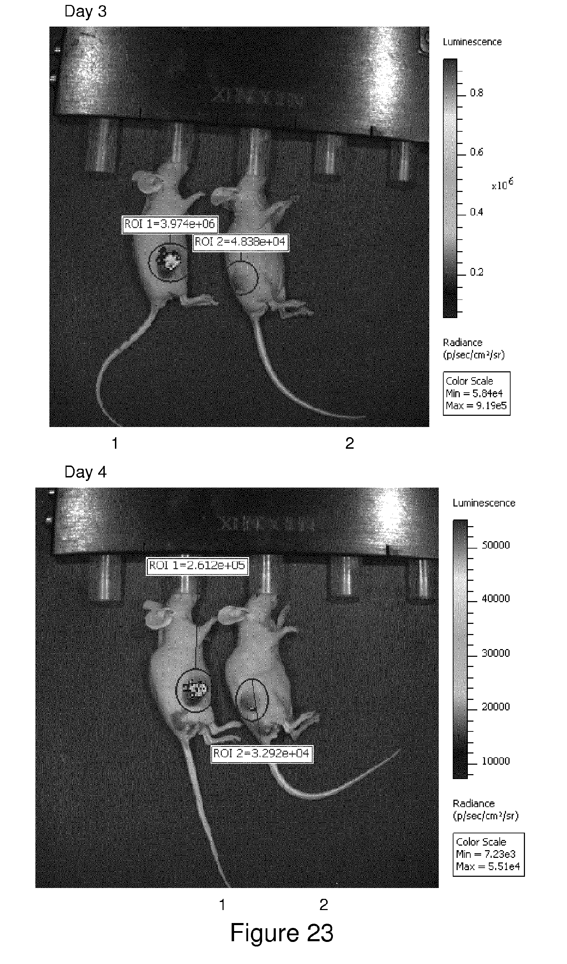

**Figure 23****.** Photographs showing luciferase expression in "dox 5" treated nude mice at day 3 and day 4 following administration.
**Figure 24****.** Charts showing light emissions of "dox 4" and "dox 5" treated mice at days 3, 4, 7 and 11 following administration.

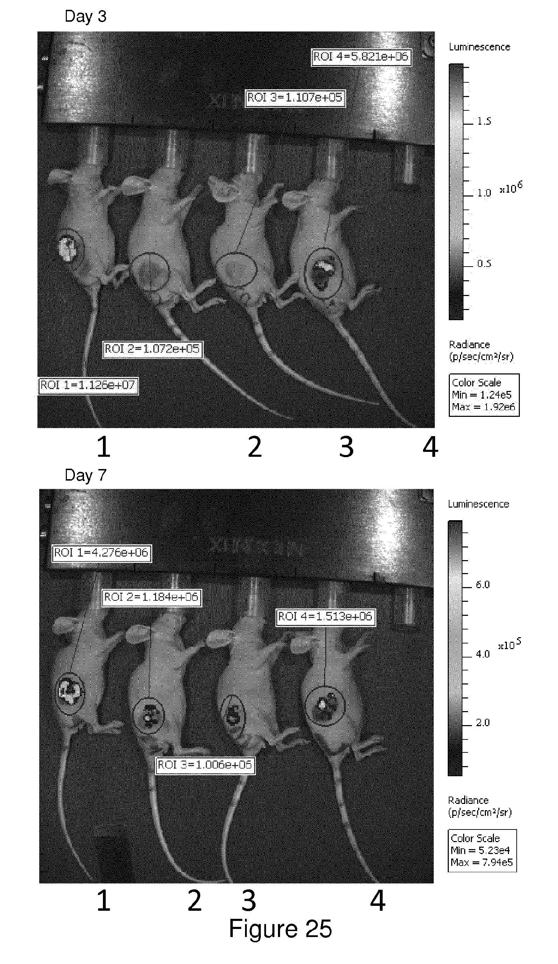

**Figure 25****.** Photographs showing luciferase expression in "dox 3" treated nude mice at day 3 and day 7 following administration.
**Figure 26****.** Chart showing light emissions of "dox 3" treated mice at days 3, 4 and 7 following administration.
**Figure 27****.** Chart showing comparison of viral titre in tumor extracts vs. survival. d1=dox 1, d2=dox 2, d3=dox 3, d4=dox 4, d5=dox 5, m1=mouse 1 etc.

### Detailed Description of the Invention

The details of one or more embodiments of the invention are set forth in the accompanying description below including specific details of the best mode contemplated by the inventors for carrying out the invention, by way of example. It will be apparent to one skilled in the art that the present invention may be practiced without limitation to these specific details.

### Examples

### Example 1 - In vitro infection of HCC lines with HSV1716

### Hepatocellular carcinoma cells

HuH-7 is a well-differentiated, hepatocyte-derived cellular carcinoma cell line that was originally taken from a liver tumor in a 57-year-old Japanese male (Vecchi et al., Hepatology 2010 Feb; 51 (2):654-9). HuH-7 are epithelial-like tumorigenic cells and are fully permissive for HSV1716

The HepG2 cell line (ATCC No. HB-8065) was derived from the liver tissue of a fifteen year old male with differentiated hepatocellular carcinoma. HepG2 are adherent, epithelial-like cells growing as monolayers and in small aggregates. HepG2 are fully permissive for HSV1716

HepG2-*luc*2 (Caliper HT1 080-*luc2*) is a luciferase expressing cell line stably transfected with the firefly luciferase gene (*luc2*). The cell line was established by transducing lentivirus containing luciferase 2 gene under the control of human ubiquitin C promoter.

### In vitro HSV1716 replication in HuH7 and HepG2-luc cells

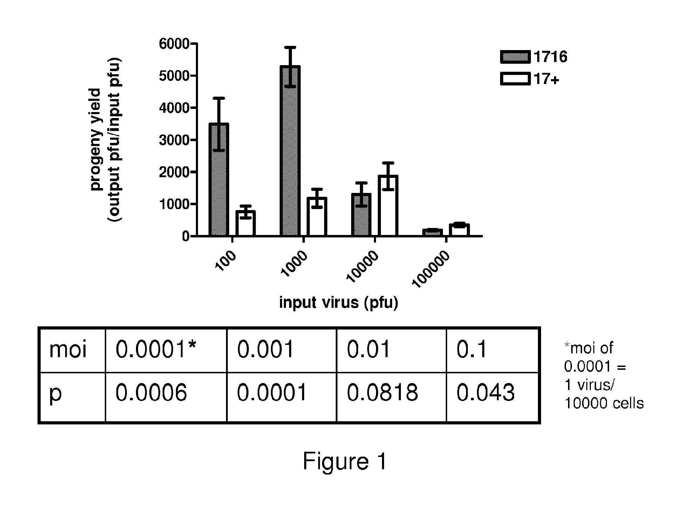

HSV1716 was found to replicate with high efficiency in HuH-7 and HepG2-luc2 cells in tissue culture (Figures 1-3).

At low multiplicities of infection (moi = number of input viruses/number of cells) in HuH-7 cells, HSV1716 replication gave higher progeny yields than parental wild-type HSV-1 17+ yields (Figure 1). Yields were similar at higher moi.

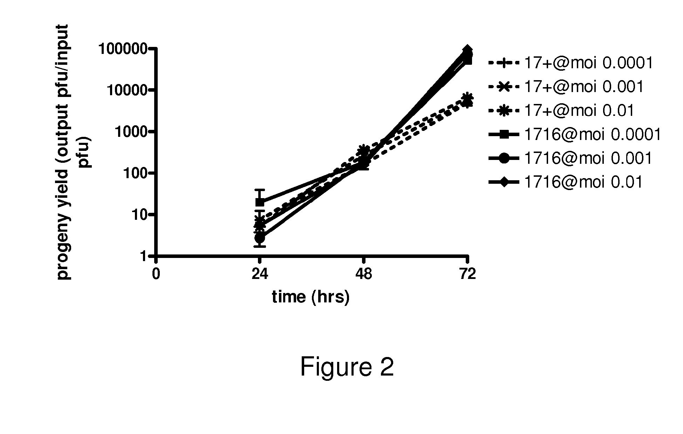

Similar levels of HSV-1 17+ and HSV1716 replication were found at 24 and 48 hrs, with a large increase in progeny production for HSV1716 between 48 and 72 hrs (Figure 2).

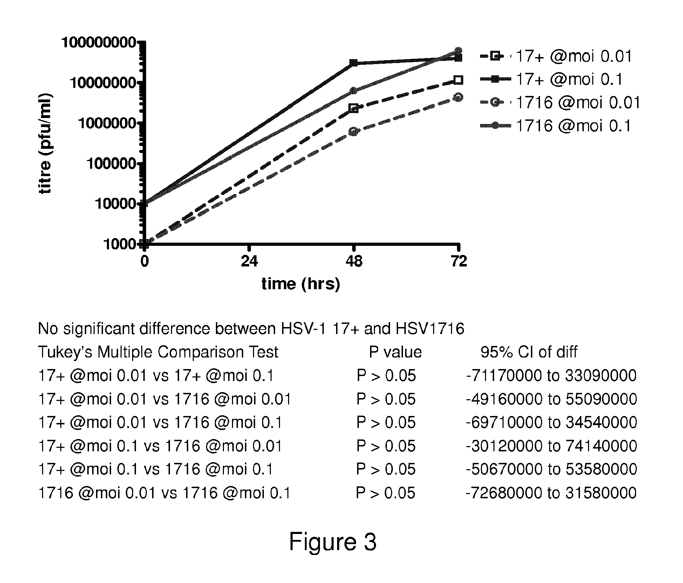

Progeny yields from HSV1716 infection of HepG2-luc2 cells were equivalent to those of wild-type HSV-1 17+, with no significant difference in yield between the viruses (Figure 3). Lu

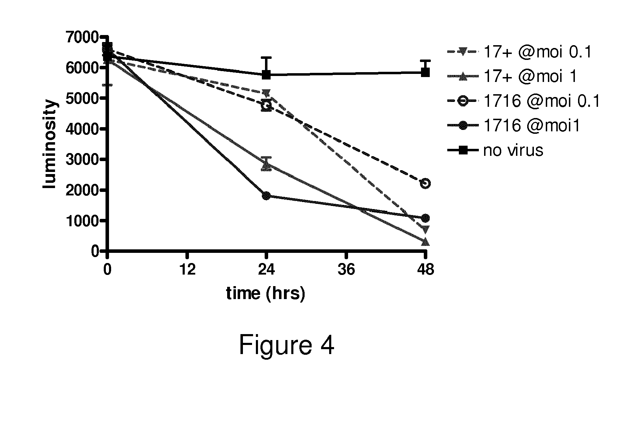

Luciferase activity decreased during HSV-1 17+ and HSV1716 infection of HepG2-luc cells (Figure 4). Efficient cell killing by virus correlates with loss of light production in HepG2-luc2 such that the loss of light output can be correlated with cell killing by HSV1716.

The results show that HuH-7 and HepG2-luc2 cells are excellent substrates for HSV1716 replication with high yields of progeny produced. HSV1716 was found to propagate in both cell types as efficiently as parental HSV-1 17+.

### Example 2 - Demonstration of HSV1716 efficacy in a Hepatocellular Carcinoma xenograft model in nude mice

### HuH-7 xenografts - biodistribution of HSV1716

HuH-7 cells were implanted subcutaneously in nude mice and allowed to form subcutaneous tumors. Subcutaneous tumors developed in 10/10 mice. All animal procedures were performed under license from the UK Home Office. Female 6-8 week old athymic nude mice (Charles River Labs, Tranent, UK) were maintained under specific pathogen free conditions and all animal experiments were performed according to the appropriate UK guidelines.

1x10⁷ pfu HSV1716 was administered to all mice via a single tail vein injection.

Tumors/organs were harvested on days 1, 4, 7, 14 and 21 post-adminstration (n=2) and biodistribution of virus was analysed by titration.

HSV1716 was found to be almost exclusively localised to tumor tissue with high titres present throughout all samples. Most other tissues/organs were found to be free of virus at all times tested. In one mouse small amounts of virus were present in the liver on day 4 and lung on day 7. This may have been the result of residual inoculum, due to blood contaminating the tissue extracts or leakage into the circulation from the excess of virus in the tumor tissue.

### HSV1716 efficacy in HuH-7 xenografts - Part 1

30 mice were injected with HuH-7 cells, yielding 22 usable subcutaneous tumors.

Mice were separated in the following groups: No virus (n= 7), 1x10⁶ pfu HSV1716 (n=7) and 1x10⁷ pfu HSV1716 (n=8), where cGMP-like HSV1716 (Virttu Biologics Limited, Glasgow, UK) was administered via tail vein injection on days 1 and 4. Tumor growth and survival was monitored in all animals until animals were sacrificed. Titration of virus in tumors was performed from sacrificed HSV1716-treated mice.

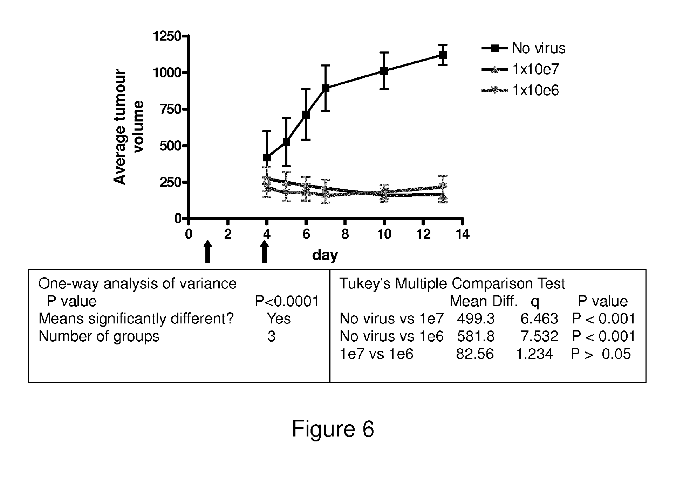

HuH7 tumors treated with HSV1716 at both doses have greatly reduced growth compared to untreated controls (Figure 6). The difference in tumor growth for untreated vs. treated was highly significant by ANOVA.

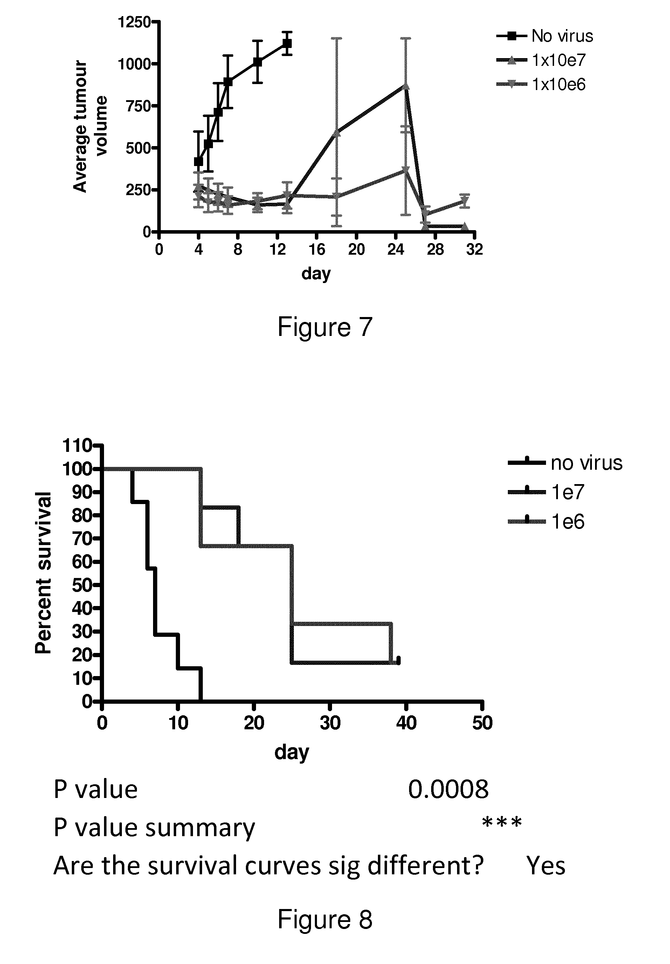

At the time of first injection there was heterogeneity in tumor sizes. Rapid tumor growth was observed after day 15 in mice which started the experiment with larger tumors (Figure 7). Beyond day 15 tumors in mice treated with 1x10⁷ pfu HSV1716 were seen to grow more rapidly, but this is considered to be due to the unequal distribution of tumor sizes at the start of the experiment with more mice with larger tumors in the 1x10⁷ group. After 28 days average tumor volume in both HSV1716 treated groups had returned to low levels (Figure 7).

The results show that intravenous HSV1716 is highly effective in treating a mouse model of HCC. Reduction in tumor growth and enhanced survival were both highly significant. High titres of HSV1716 in tumor extracts indicate prolific replication of virus in tumor cells. The lower dose of HSV1716 (1x10⁶ pfu) was at least as effective as the higher dose (1x10⁷ pfu)

### HSV1716 efficacy in HuH-7 xenografts - Part 2

Mice were injected with HuH-7 cells to generate subcutaneous tumor xeongrafts, as described above.

Mice were separated in the following groups: No virus (n= 5), 1x10⁵ pfu HSV1716 (n=6), 1x10⁶ pfu HSV1716 (n=6), and 1x10⁷ pfu HSV1716 (n=6) where cGMP-like HSV1716 (Virttu Biologics Limited, Glasgow, UK) was administered via tail vein injection on days 1, 14 and 29. Tumor growth and survival was monitored in all animals until animals were sacrificed. Titration of virus in tumors was performed from sacrificed HSV1716-treated mice.

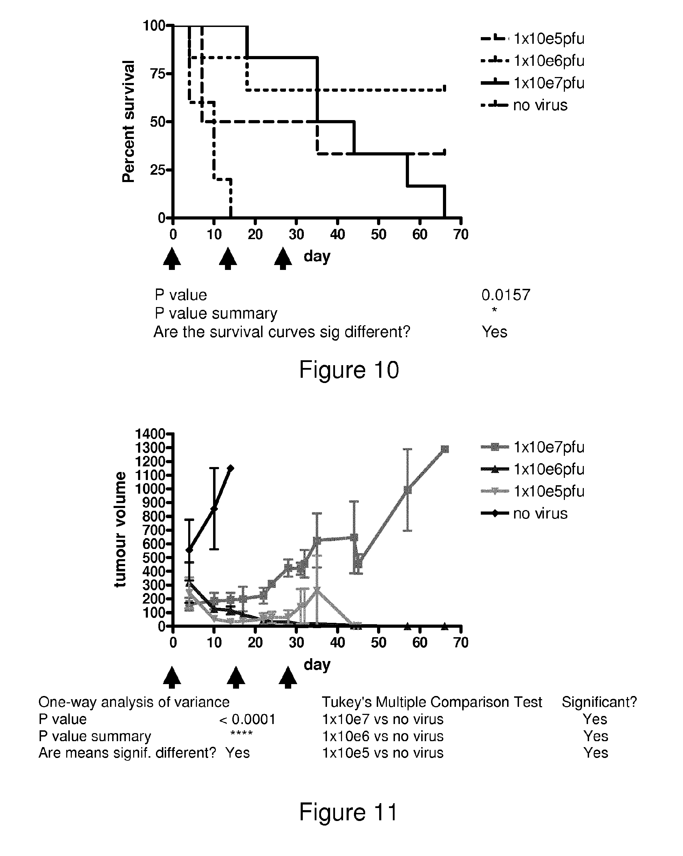

The results show that all control mice (injected with PBS only) were sacrificed by day 14 and that mice injected with HSV1716 had greatly improved survival (Figure 10). On day 66, when the experiment was stopped, 2/6 mice treated with 1x10⁵ pfu were cured, 4/6 mice treated with 1x10⁶ pfu were cured but there were no cures in 1x10⁷ pfu group.

Analysis of data from individual mice indicates that mice with large tumors at time of injection exhibited a poorer survival (3/3 receiving 1x10⁵ pfu HSV1716, 1/3 receiving 1x10⁶ pfu HSV1716 within 7 days) compared to those with smaller xenografts. Initially mice with larger tumors receiving 1x10⁷ pfu HSV1716 had slower growth and survived longer than mice with similar size tumors receiving 1x10⁵ HSV1716 and 1x10⁶ HSV1716 pfu but there were no cures and these tumors continued to grow. Most mice with smaller tumors at the time of first administration of 1x10⁵ or 1x10⁶ pfu HSV1716 were cured (5/6). Cure rates were higher in the 1x10⁶ group than in the 1x10⁵ group. No virus was found at the xenografts sites in cured mice.

The results show that intravenous administration of HSV1716 is treating HCC tumor xenografts. The reduction in tumor growth and enhanced survival were both highly significant. Very high titres of HSV1716 in tumor extracts indicate prolific replication of virus. 50% of mice were cured with 3 repeat doses at 14 day intervals. A dose of 1x10⁵ pfu HSV1716 was at least as effective as 1x10⁶ pfu HSV1716.

### Example 3 - Infection of HuH7 xenografts by HSV1716 after intravenous administration

Mice were injected with HuH-7 cells to generate subcutaneous tumor xeongrafts, as described above.

### Experiment 1

HSV1716 (1x10⁷ pfu) was administered via tail vein injection to 4 mice with HuH7 xenografts. Mice were sacrificed 16, 24, 48 and 72 hours after HSV1716 injection. Tumors were extracted and virus titrated.

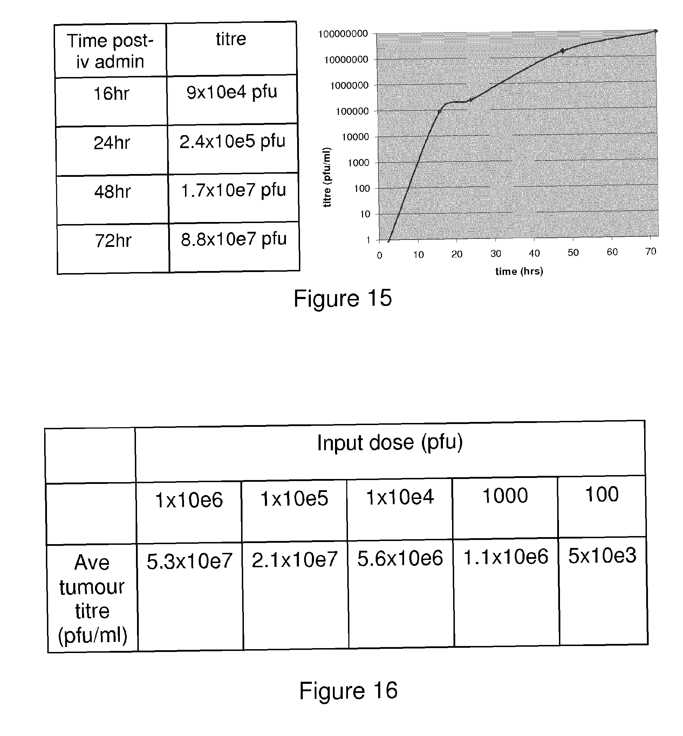

After 16 hours 1% of the input HSV1716 was seen to have localised to cells of the HuH-7 xenograft tumor (Figure 15). Within 24 hours a 100-fold increase in HSV1716 within cells of the tumor was observed (Figure 15). The HSV1716 replication kinetics were seen to be similar to those observed in vitro (Figure 15).

### Experiment 2

Mice (n=2) with HuH-7 xeongraft tumors were intravenously injected with increasing doses of HSV1716 (100, 1000, 1x10⁴, 1x10⁵, 1x10⁶ pfu). Tumors were harvested 72 hours after injection and analysed by titration.

Highly efficient localisation of HSV1716 to HuH-7 xenografts was observed, even at the lowest doses (Figure 16). Rapid replication of virus within the xenografts was also observed.

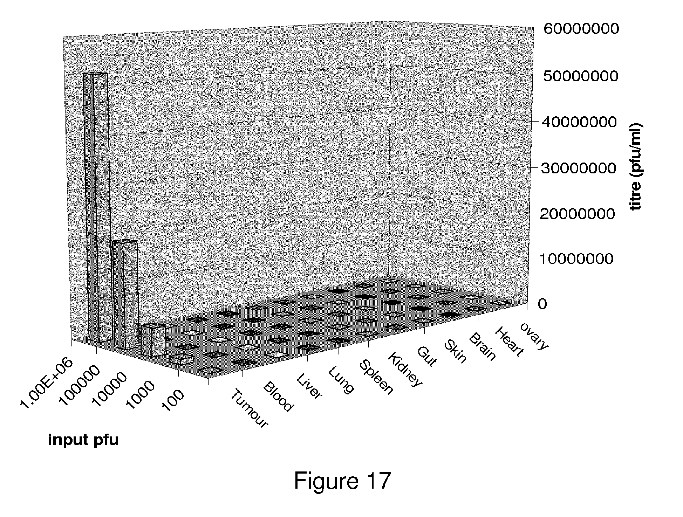

A biodistribution study showed HSV1716 to be present in tumor tissue only (Figure 17).

### Example 4 - HSV1716 in combination with doxorubicin in vivo

We investigated whether there is any loss of viral potency when HSV1716 is administered in combination with doxorubicin.

### Materials

- Mice with HuH-7 xenografts, as described above.
- HSV1716luc - a mutant of HSV1716 expressing the luciferase reporter gene.
- Doxorubicin salt (Caliper Life Sciences, Inc.)

By constructing an HSV1716 that conditionally expresses luciferase on viral replication, localisation of active virus in vivo can be detected by imaging for luminosity as a result of luciferase acting on the substrate luciferin.

### Methods

Nude mice with subcutaneous flank HuH-7 xenografts were injected with virus once the xenograft diameters were approximately 5 mm. All animals received 2x10⁶ pfu HSV1716luc.

Doxorubicin salt (specifically formulated for use in *in vivo* imaging) was made up in water at the time of injection. 10mg was resuspended in 5ml of water = 2mg/ml. 150µl was injected per mouse = 0.3mg. Mice weighed approximately 25g, thus equivalent dose is 12mg/kg. Equivalent to 36mg/m² (human dose is typically 40-60mg/m²).

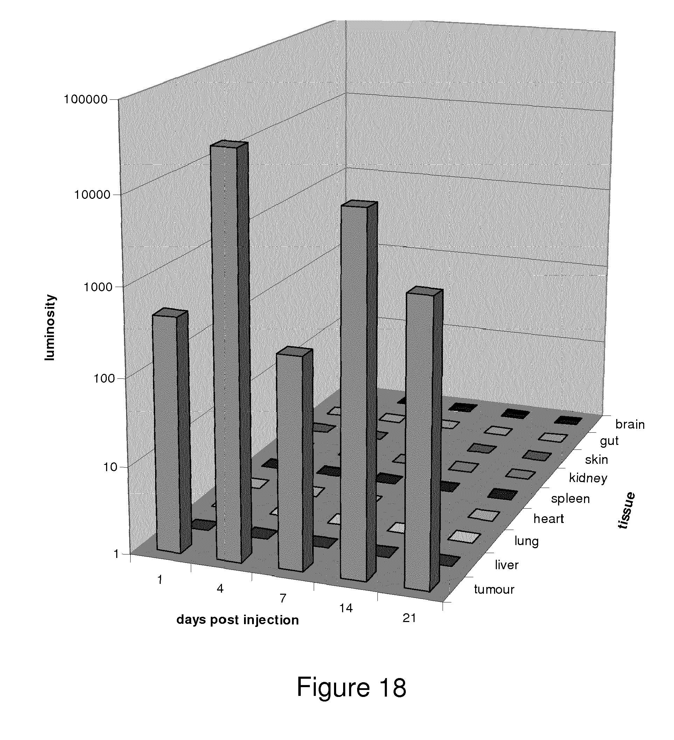

Following intravenous injection of 1x10⁷ pfu HSV1716luc into mice having subcutaneous HuH-7 xenograft luceiferase expressionwas detected by invivo imaging analysis 72 hours after injection. Luciferase expression was found to be uniquely localised to tumor xeongraft tissue, which is consistent with the biodistribution studies described in the previous examples. Luciferase expression reminaed localised to xenograft tumors (although of reduced size) after 21 days following injection (Figure 18).

### Experiment 1

Comparison of effect of:
1. 36mg/m² Doxorubicin mixed with 2x10⁶ pfu HSV1716luc injected intratumorally ("dox 1 ") and
2. PBS mixed with 2x10⁶ pfu HSV1716luc and injected intratumorally ("dox 2")

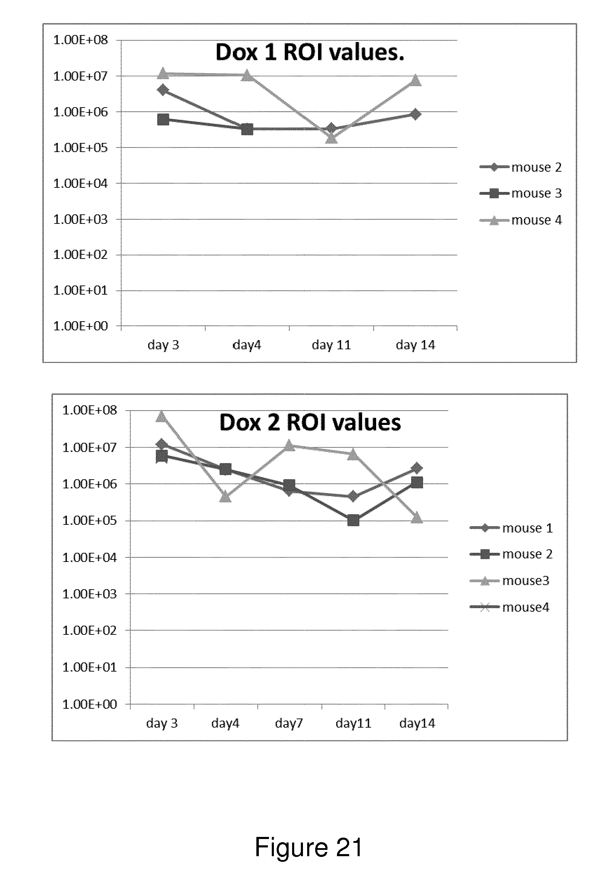

Light emissions from tumors injected with "dox 1" and "dox 2" were measured at days 3, 4, 11 and 14. Light emissions were similar for both groups (Figure 21) indicating that Doxorubicin has no apparent effect on HSV1716 replication following simultaneous intratumoral administration.

### Experiment 2

Comparison of effect of:
1. 2x10⁶ pfu HSV1716luc was injected intravenously ("dox 4"), and
2. 36mg/m² Doxorubicin was injected intratumorally, 2x10e6pfu HSV1716luc was injected intravenously ("dox 5")

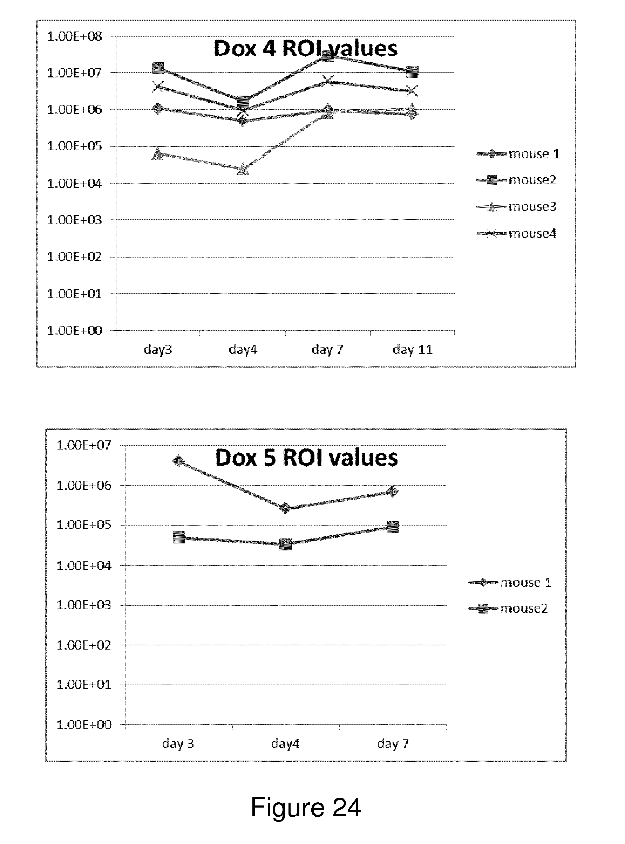

Light emissions from tumors injected with "dox 1" and "dox 2" were measured at days 3, 4, 7 and 11. Light emissions were similar for both groups (Figure 24) indicating that Doxorubicin has no apparent effect on HSV1716 replication following intravenous administration of HSV1716 and intratumoral administration of Doxorubicin.

### Experiment 3

36mg/m² Doxorubicin was injected intraperitoneally, and 2x10⁶ pfu HS1716luc was injected intratumorally ("dox 3").

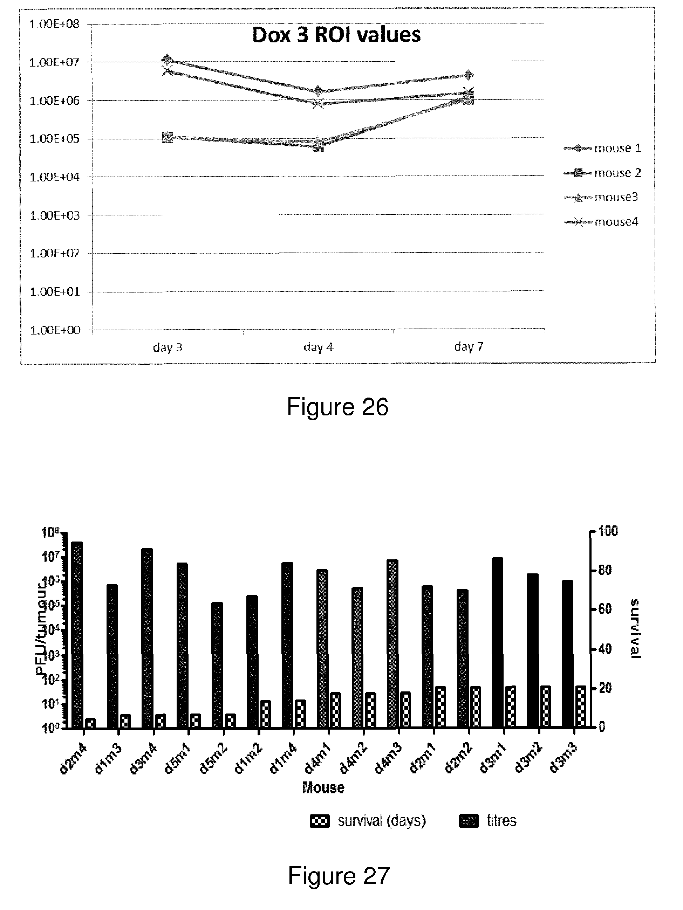

We observed that Doxorubcin injected intraperitoneally does not block replication of intratumoral HSV1716 (Figure 26).

For experiments 1-3 above, viral titre in tumor extracts was compared against survival (Figure 27). High virus titres were observed in all tumor extracts indicating that simultaneously administered doxorubicin does not affect HSV1716 replication in vivo.

### Example 5 - Phase I/II study of HSV1716 in HCC

A phase I/II study will be conducted investigating the safety, tolerability and biological effect of single and repeat intra-arterial injections of the selectively replication-competent herpes simplex virus HSV1716 in patients with unresectable hepatocellular carcinoma.

The study will comprise a dose escalation component. Delivery of virus will be into the hepatic artery using the Seldinger technique. CT scans will be conducted at baseline, day 28 and day 56. A biopsy will be taken at day 28 - one core from a lesion, one core from normal tissue.

### Stage 1

The primary objective of Stage 1 will be to assess the safety and tolerability of HSV1716 given by direct intra-arterial injection with TACE in patients with unresectable hepatocellular carcinomas.

The secondary objectives will be:
- to compare the safety information from Stage 1 with matched historical data from TACE
- to assess tumor response by measurement of tumor size by CT and the tumor marker α-FP
- to assess evidence of biological activity in pre- and post-treatment biopsy samples
- to assess the immune response to HSV1716 by measurement of circulating anti-HSV IgG and IgM
- to determine whether HSV1716 can be detected by PCR within the peripheral circulation after intra-arterial injection

### Stage 1

Stage 1 will be conducted at a single-centre, have an ascending dose design, and be open label. During their hepatic arteriography and prior to receiving the chemoembolisation agents, patients will receive a single intra-arterial injection of HSV1716 according to the following dosing regime:

| | Dose |
|---|---|
| Group 1 (3 patients) | 1 x 10⁶ pfu |
| Group 2 (3 patients) | 4 x 10⁶ pfu |
| Group 3 (3 patients) | 1 x 10⁷ pfu |

Delivery will be carried out during the TACE procedure by intra-arterial injection into a branch of the main hepatic artery supplying the tumor(s) and prior to the delivery of the TACE agents (typically, doxorubicin, lipiodol(contrast agent and vehicle for doxorubicin delivery into the tumor bed) and embolisation agent (for subsequent blockage of the tumor-feeding artery)).

### Stage 2

Stage 2 will be conducted at a single-centre, and have a randomised, controlled, two-arm open label study design.

30 patients will be randomised in a 2:1 ratio to receive HSV1716 plus TACE or TACE alone. In the treatment arm, patients will receive two doses of HSV1716 by intra-arterial injection. The optimal dose will be determined in Stage 1. The first dose will be administered during the TACE procedure. A 2 week interval between the first and second intra-arterial administration will be provided. In the control arm, patients will receive TACE as per standard of care.

### Example 6 - HSV1716 compatibility with delivery via TACE catheter and Doxorubicin (chemotherapeutic agent for TACE)

The aim of this experiment was to establish if there is any loss of viral titre using the materials and administration method to be used in the Phase I/II study described in Example 5 above.

### Materials

- Progreat Coaxial type catheter 2.7/2.9 fr, product code MC-PP27131.
- Boston scientific Floswitch Ref 44-200, UPN M001442000.
- CRU-01-A vial 47 1716 HSV1 2 x 10⁶ iu/ml (0.5ml used = 1x10⁶ iu)

### Methods

1. Progreat Coaxial type catheter and Boston scientific Floswitch are assembled in the class II hood
2. Pre-flush catheter with sterile saline (Baxters UKF 7114) to determine dead volume.
3. Transfer catheter tip to collection tube 1, 0.5ml of CRU-01-A is passed through the catheter using a 1 ml luer syringe (Becton Dickinson ref 300912).
4. 5ml syringe (Becton Dickinson ref 302187) containing sterile saline is attached to floswitch.
5. A volume equal to dead volume of catheter is collected in tube 1 (dead volume).
6. The catheter tip is transferred to tube 2 and the next 0.5ml passed through the catheter is collected (virus flow through).
7. Transfer catheter tip to tube 3 and the next 0.5ml passed through the catheter is collected (post virus).
8. Transfer catheter tip to tube 4 and the remainder of the 5.0ml saline is passed through the catheter and collected (remaining NaCl).

| Tube | Vol (ml) | Titre (pfu/ml) | Sample type | TOTAL VIRUS OFF (pfu) | TOTAL VIRUS ON (pfu) |
|---|---|---|---|---|---|
| 1 | 0384 | 3.69x10⁵ | Dead volume | 2.52x10⁵ | |
| 2 | 0.551 | 1.01x10⁶ | Virus flow through | 5.57x10⁵ | |
| 3 | 0.906 | 5.40x10⁴ | 0.5ml post virus | 4.89x10⁴ | |
| 4 | 3.287 | 9.75x10² | Remaining NaCl | 3.20x103 | |
| | | | | **8.61x10⁵** | **9.7x10⁵** |

### Conclusion

From the data collected in this study, there was no significant reduction in viral titre using the equipment and delivery process.

Thus HSV1716 is fully compatible with the administration procedure and the TACE catheter.

## Claims

1. HSV1716 for use in a method of treating hepatocellular carcinoma in a human patient, the method comprising intra-arterial administration of HSV1716 to the hepatic artery.

2. HSV1716 for use in a method of treating hepatocellular carcinoma in a human patient according to claim 1, wherein the method further comprises simultaneous or sequential intra-arterial administration of doxorubicin to the hepatic artery or oral administration of sorafenib.

3. An oncolytic herpes simplex virus for use in a method of treating primary liver cancer, the method comprising intra-arterial or intravenous administration of an oncolytic herpes simplex virus.

4. An oncolytic herpes simplex virus for use in a method of treating primary liver cancer according to claim 3, wherein the method further comprises administration of doxorubicin or sorafenib.

5. An oncolytic herpes simplex virus for use in a method of treating primary liver cancer according to claim 3, wherein the method further comprises simultaneous or sequential intra-arterial or intravenous administration of doxorubicin and/or oral administration of sorafenib.

6. An oncolytic herpes simplex virus for use in a method of treating primary liver cancer according to any one of claims 3 to 5, wherein administration of the oncolytic herpes simplex virus is to the hepatic artery.

7. An oncolytic herpes simplex virus for use in a method of treating primary liver cancer according to claim 5, wherein administration of the oncolytic herpes simplex virus and doxorubicin is to the hepatic artery.

8. An oncolytic herpes simplex virus for use in a method of treating primary liver cancer according to any one of claims 3 to 7, wherein the primary liver cancer is hepatocellular carcinoma.

9. An oncolytic herpes simplex virus for use in a method of treating primary liver cancer according to any one of claims 3 to 8, wherein all copies of the ICP34.5 gene in the genome of the oncolytic herpes simplex virus are modified such that the ICP34.5 gene is incapable of expressing a functional ICP34.5 gene product.

10. An oncolytic herpes simplex virus for use in a method of treating primary liver cancer according to any one of claims 3 to 9, wherein the oncolytic herpes simplex virus is a mutant of HSV-1 strain 17.

11. An oncolytic herpes simplex virus for use in a method of treating primary liver cancer according to any one of claims 3 to 8, wherein the oncolytic herpes simplex virus is HSV1716.

12. A pharmaceutical composition comprising an oncolytic herpes simplex virus and a chemotherapeutic agent, wherein the chemotherapeutic agent is doxorubicin or sorafenib.

13. A pharmaceutical composition according to claim 12, wherein the oncolytic herpes simplex virus is HSV1716.

14. A kit comprising a predetermined amount of oncolytic herpes simplex virus and a predetermined amount of chemotherapeutic agent, wherein the chemotherapeutic agent is doxorubicin or sorafenib.

15. Products containing therapeutically effective amounts of:
(i) HSV1716, and
(ii) Doxorubicin or Sorafenib
for simultaneous or sequential use in a method of medical treatment, preferably treatment of primary liver cancer.
